# EUROPEAN PATENT APPLICATION

(11) **EP 4 001 395 A2**
(43) Date of publication of application: **25.05.2022**
(21) Application number: 21205783.0
(22) Date of filing: 01.11.2021
(51) Int. Cl.: C12M 1/22, C12M 3/00, C12M 1/00, C12M 1/34, G01N 35/02

(54) **A SYSTEM AND A METHOD FOR AUTOMATIC MANAGEMENT OF ORGANIC SAMPLE(S)**

(30) Priority: 02.11.2020 IN 202021047691
(71) Applicant: Airamatrix Private Limited, 400604 Maharashtra (IN)
(72) Inventor: SINGHAL, Nitin, 560067 Bangalore (IN); BHARATHI, Vijay, 400607 Thane-West (IN); KONDRAGUNTA, Satya, Chaitanya, 400012 Mumbai (IN)
(74) Representative: Budde Schou A/S

(57) **Abstract**

The embodiments herein generally relate to a micro-biological colony counters and more particularly, to a system and a method for feeding, identifying, counting, classifying, segregating and collecting organic samples such as but not limited to micro-organisms. The system can classify colonies in different classes such as bacteria and fungus present in an organic sample and also gives a digital count on the number of colonies present in each of the classes separately. The system reduces the time in counting and classification of microbes in each class separately, eliminates manual errors and requires less manual intervention. The system is reliable and can perform the counting and classification of microbes in each class separately even in absence of well-trained technician. The system can count surface colonies in petri-dish and can count colonies in different size or diameter of petri-dish.

## Description

### TECHNICAL FIELD

The embodiments herein generally relate to micro-biological colony counters and more particularly, to a system and a method for automatic feeding, identifying, counting, classifying, segregating and collecting organic samples such as but not limited to micro-organisms.

### BACKGROUND

Microbiology is the study of microscopic organisms such as bacteria, algae, viruses, archaea, fungi and protozoa. Microbiology includes fundamental research on the biochemistry, physiology, cell biology, ecology, evolution and clinical aspects of the micro-organisms. Further, microbiology research includes the formation of colonies of micro-organisms such as bacteria on a growth medium such as agar which is disposed on a petri dish. The microbial colonies are manually counted by a lab technician using microscope devices, wherein the count of the individual colonies is used to determine the effectiveness of various chemicals. Such colony counting is performed in laboratory work, bio-medical facilities and also in pharmaceutical industry. For example, the number of organisms in a blood agar may be counted in a research laboratory or a physician may make a culture of an infections organism during an examination. Further, in quality control of food and beverage industries, the number of micro-organisms present in a product must be regularly checked. Also, in pharmaceutical industry the number of micro-organisms in clean room setting must be checked regularly and in compliance with the regulatory norms.

Colony counting within a culture plate involves many number of culture plate transport which includes moving culture plates from incubators to microscope plate and to storage back again. Manual counting of the bacteria colonies is difficult especially for a novice and hence, requires trained laboratory technician. Manual counting of the bacteria colonies is time consuming and involves relatively high labor costs. Additionally, the manual counting of colonies by the lab worker may also results in inaccurate counts of the bacteria colonies. For example, in some instances up to one thousand colonies can be counted and such colonies may be as small as 0.1 millimeters and spaced as close as 0.2 millimeters. As a result, such counting is extremely time consuming, inaccurate, laborious, and exceedingly costly both in time and required skilled labor. Typically, 80% of the petri-dish in a single batch does not have any colony growth. In an analog system, the microbiologist needs to analyze all the petri-dishes with or without colony growth which is time consuming and laborious resulting in fatigue to the lab technician.

Therefore, there exists a need for a system for automatic feeding, identifying, counting, classifying, segregating and collecting organic samples. Further, there exists a need for a system and a method for automatic management of organic samples, which obviates the aforementioned drawbacks.

### OBJECTS

The principal object of embodiments herein is to provide a system for automatic management of organic samples such as but not limited to micro-organisms.

Another object of embodiments herein is to provide a system for automatic feeding, identifying, counting, classifying, segregating and collecting organic samples such as but not limited to micro-organisms

Another object of embodiments herein is to provide a method for automatic feeding, identifying, counting, classifying, segregating and collecting organic samples such as but not limited to micro-organisms.

Another object of embodiments herein is to provide a modular system for at least one of automatic feeding, identifying, counting, classifying segregating and collecting managing organic samples.

Another object of embodiments herein is to provide a compact automated colony counter system which consumes less space in the room.

Another object of embodiments herein is to provide a system with a color calibrated scanner device for automatic lighting, conditioning and capturing accurate images of organic sample(s) cultivated on petri dishes of different sizes, where the scanner device is configured for use in colony counting of micro-organisms.

Another object of embodiments herein is to provide a system with a scanner device which is configured for automated entry and exit of the organic sample(s) for point accuracy, the scanner device which is adapted to sense the presence of the petri-dish inside a photo compartment and automatically triggers the scan acquisition control.

Another object of embodiments herein is to provide a system with a scanner device for standardizing the imaging process by eliminating entrance of any ambient light by providing a closed photo compartment at all times thereby enhancing the quality of image captured by the device resulting in reliable colony counting.

Another object of embodiments herein is to provide an automated colony counter system which can classify colonies in different classes such as bacteria and fungus present in an organic sample and also gives a digital count on the number of colonies present in each of the classes separately.

Another object of embodiments herein is to provide a micro-biological colony counter which can display the scanned image of organic sample together with a dot or contour or bounding box automatically superimposed over each individual colony that has been counted and also displays color coding based on the class of micro-organism detected.

Another object of embodiments herein is to provide a micro-biological colony counter which provides an output on a digital count of the number of colonies such that the output can further be re-classified into different classes of micro-organisms by a trained technician.

Another object of embodiments herein is to provide a micro-biological colony counter which can count surface colonies in petri-dish and can count colonies in different size or diameter of petri-dish.

Another object of embodiments herein is to provide an automated micro-biological colony counter which reduces the time in counting and classification of microbes in each class separately, eliminates manual errors and requires less manual intervention.

Another object of embodiments herein is to provide an automated micro-biological colony counter which is accurate, reliable and can perform the counting and classification of microbes in each class separately even in absence of well-trained technician.

These and other objects of embodiments herein will be better appreciated and understood when considered in conjunction with following description and accompanying drawings. It should be understood, however, that the following descriptions, while indicating embodiments and numerous specific details thereof, are given by way of illustration and not of limitation. Many changes and modifications may be made within the scope of the embodiments herein without departing from the spirit thereof, and the embodiments herein include all such modifications.

### BRIEF DESCRIPTION OF DRAWINGS

The embodiments are illustrated in the accompanying drawings, throughout which like reference letters indicate corresponding parts in various figures. The embodiments herein will be better understood from the following description with reference to the drawings, in which:
Fig. 1a depicts a perspective view of a system for feeding, identifying, counting, classifying, segregating and collecting organic samples, according to embodiments as disclosed herein;
Fig. 1b depicts another perspective view of the system showing a petri dish feeding system, according to embodiments as disclosed herein;
Fig. 2 depicts a perspective view of the petri dish feeding system, according to embodiments as disclosed herein;
Fig. 3 depicts another perspective view of the petri dish feeding system, according to embodiments as disclosed herein;
Fig. 4a depicts a perspective view of a petri dish feeding device of the petri dish feeding system, according to embodiments as disclosed herein;
Fig. 4b depicts a perspective view of a petri dish locking device of the petri dish feeding system, according to embodiments as disclosed herein;
Fig. 4c depicts another perspective view of the petri dish feeding system, according to embodiments as disclosed herein;
Fig. 4d depicts a cross sectional view of the petri dish feeding system, according to embodiments as disclosed herein;
Fig. 4e depicts a perspective view of geneva and geneva cam of the petri dish feeding device, according to embodiments as disclosed herein;
Fig. 5a depicts a perspective view of a temperature regulating system, according to embodiments as disclosed herein;
Fig. 5b depicts an exploded view showing a petri dish identifying system and a petri dish rejecting system, according to embodiments as disclosed herein;
Fig. 6a depicts a perspective view the petri dish rejecting system in which a flap assembly is in an open position, according to embodiments as disclosed herein;
Fig. 6b depicts a perspective view the petri dish rejecting system in which the flap assembly is in a closed position, according to embodiments as disclosed herein;
Fig. 7 depicts a petri dish conveying system, according to embodiments as disclosed herein;
Fig. 8a depicts a perspective view of a geneva mechanism of the petri dish conveying system, according to embodiments as disclosed herein;
Fig. 8b depicts a perspective view showing a position sensor installed on a bottom side of a petri dish conveying member of the petri dish conveying system, according to embodiments as disclosed herein;
Fig. 8c depicts a perspective view showing the petri dish conveying member of the petri dish conveying system, according to embodiments as disclosed herein;
Fig. 9 depicts an exploded view of a scanner device for lighting, conditioning and capturing image(s) of organic sample(s), according to embodiments as disclosed herein;
Fig. 10 depicts a perspective view of the scanner device, according to embodiments as disclosed herein;
Fig. 11 depicts a cross-sectional view of the scanner device, according to embodiments as disclosed herein; and
Fig. 12a depicts a perspective view of a petri dish sorting and collection system, according to embodiments as disclosed herein;
Fig. 12b depicts a front view of the petri dish sorting and collection system, according to embodiments as disclosed herein;
Fig. 13a a depicts a perspective view of proximity sensors located in vicinity of scanner device, according to embodiments as disclosed herein;
Fig. 13b depicts a perspective view of a petri dish conveying member of the petri dish conveying system, according to embodiments as disclosed herein; and
Fig. 14 depicts a flowchart a method for automatic management of organic samples, according to embodiments as disclosed herein.

### DETAILED DESCRIPTION

The embodiments herein and the various features and advantageous details thereof are explained more fully with reference to the non-limiting embodiments that are illustrated in the accompanying drawings and detailed in the following description. Descriptions of well-known components and processing techniques are omitted so as to not unnecessarily obscure the embodiments herein. The examples used herein are intended merely to facilitate an understanding of ways in which the embodiments herein may be practiced and to further enable those of skill in the art to practice the embodiments herein. Accordingly, the examples should not be construed as limiting the scope of the embodiments herein.

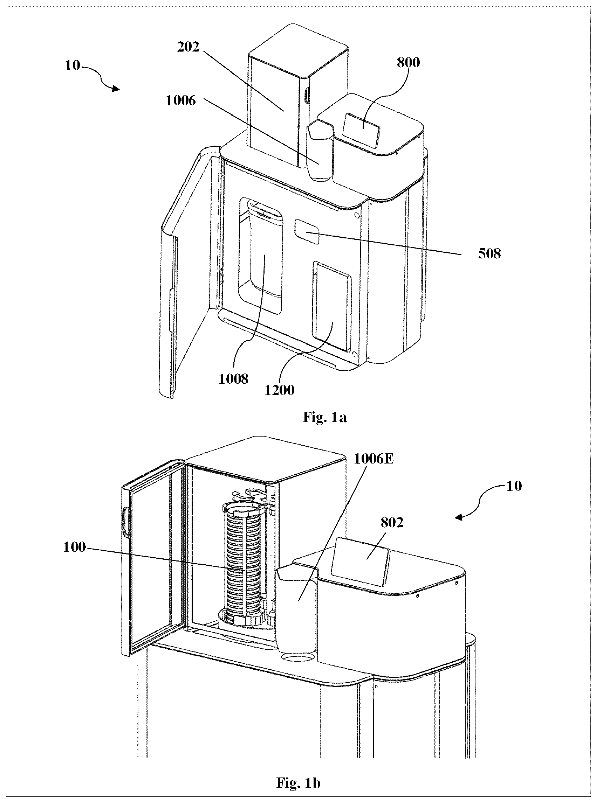

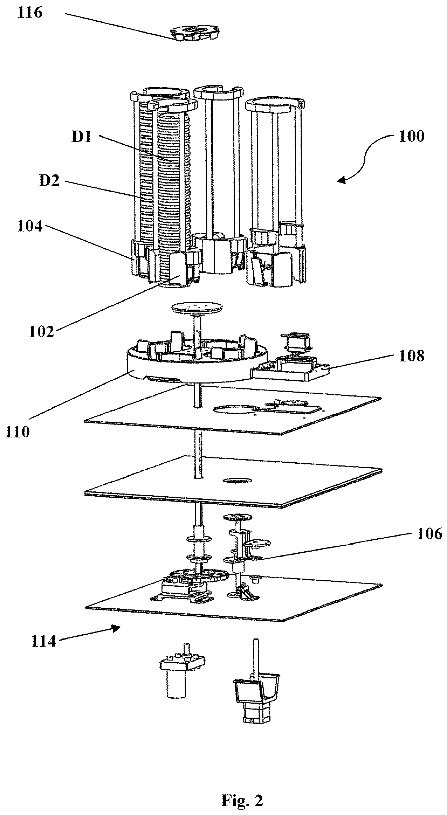

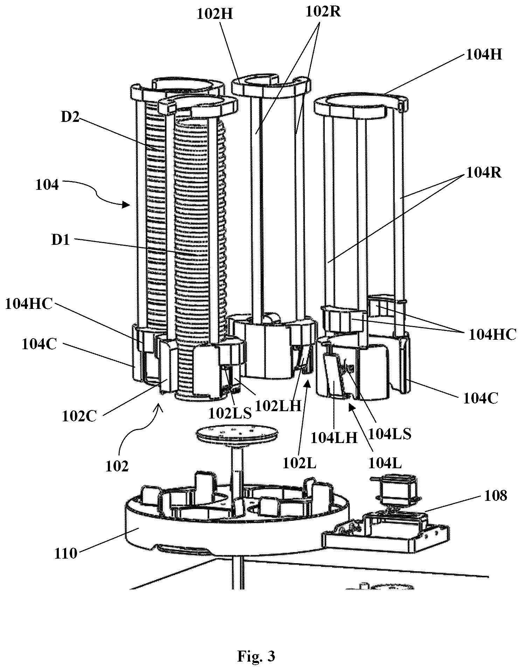

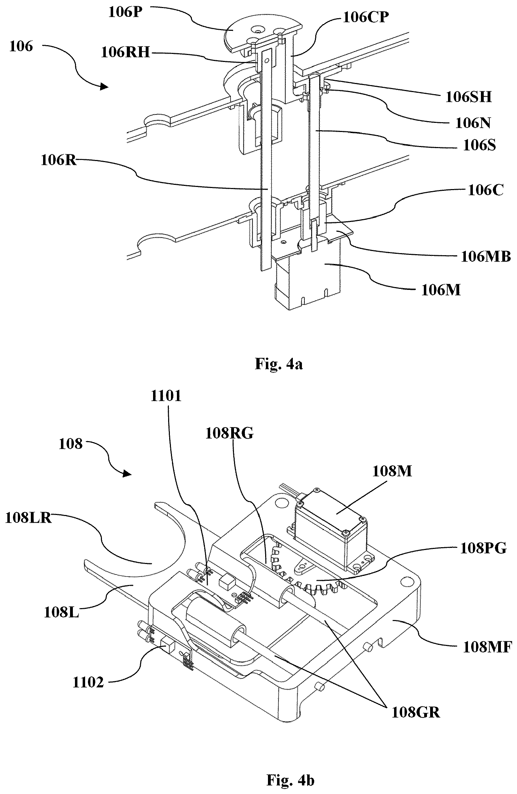

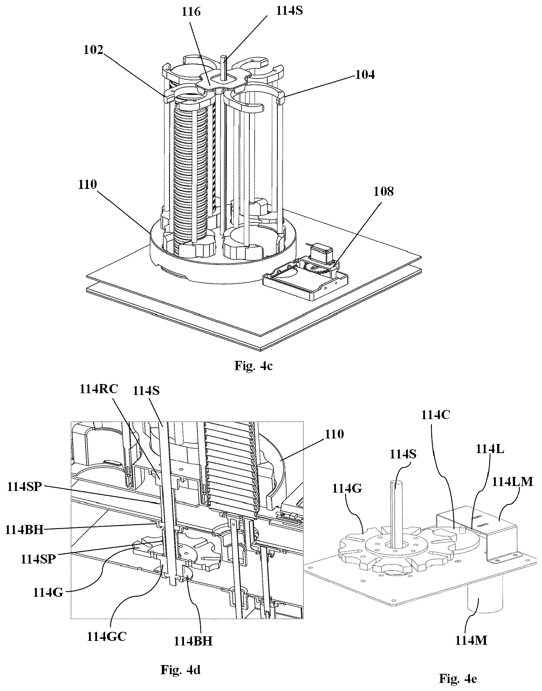

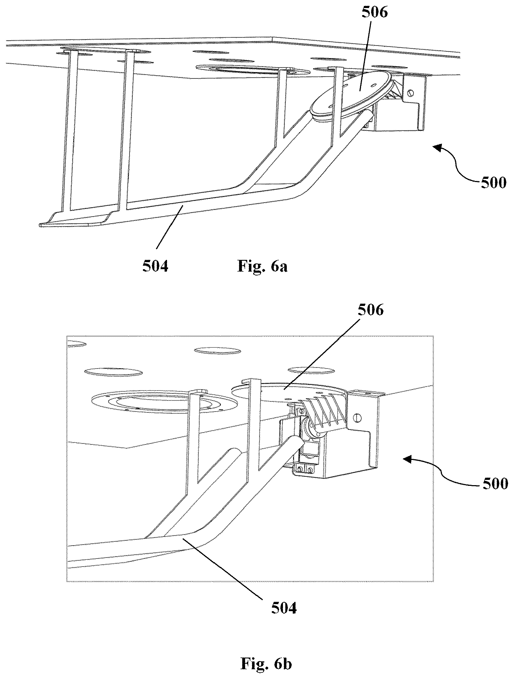

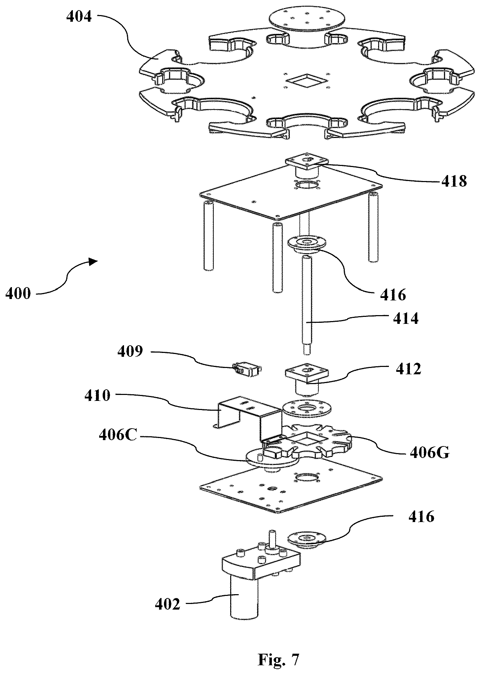

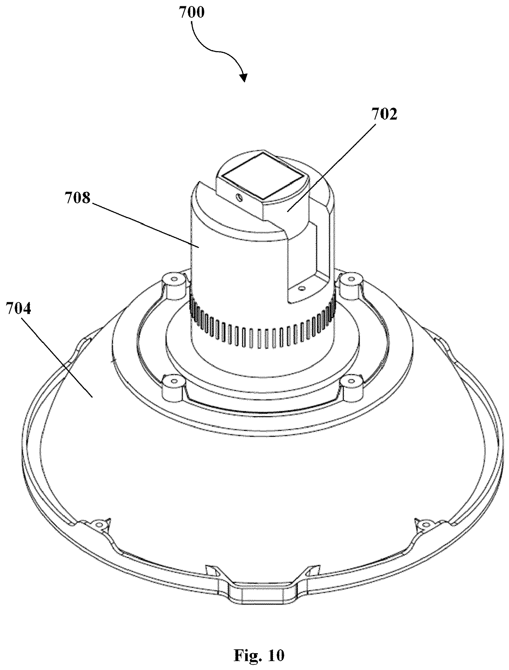

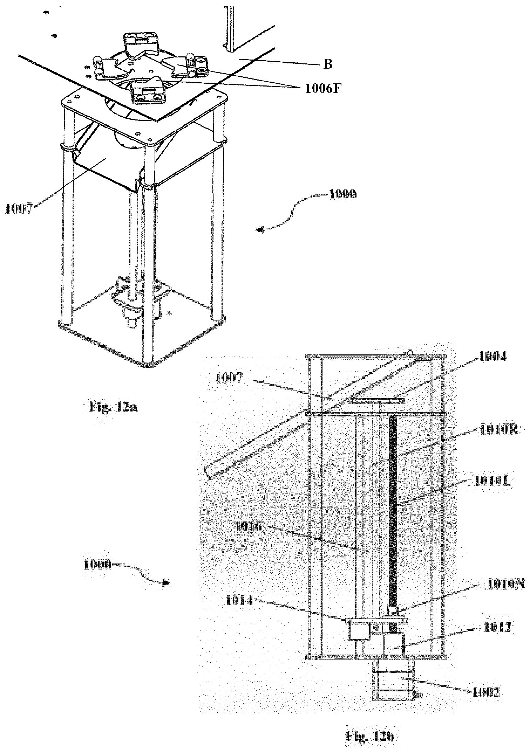

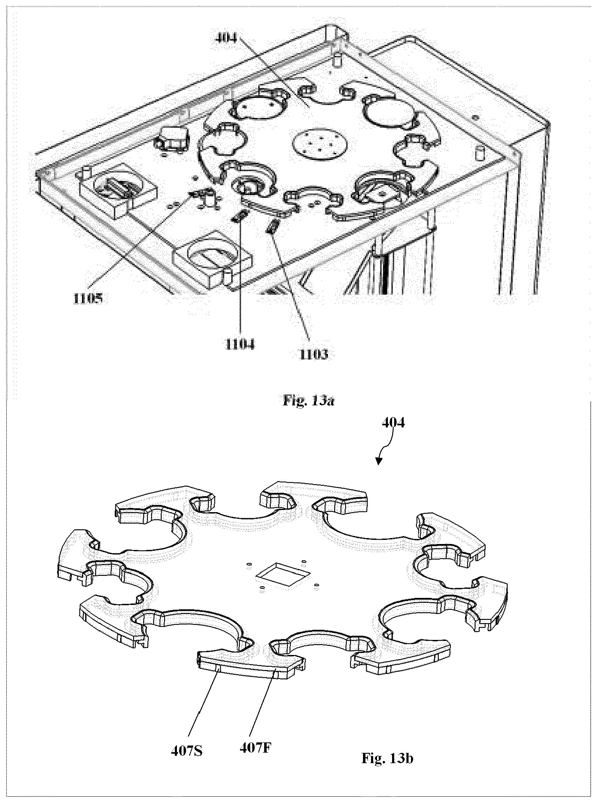

The embodiments herein achieve a system and a method for automatic feeding, identifying, counting, classifying, segregating and collecting organic samples such as but not limited to micro-organisms. Referring now to the drawings Figs 1 through 14, where similar reference characters denote corresponding features consistently throughout the figures, there are shown embodiments.

Fig. 1 depicts a perspective view of a system (10) for automatic feeding, identifying, counting, classifying, segregating and collecting organic samples, according to embodiments as disclosed herein. In an embodiment, the system (10) includes a petri dish feeding system (100), a temperature regulating system (200), a petri dish identification system (300), a petri dish conveying system (400), a petri dish rejecting system (500), a scanner device (700), a user interface unit (800), a petri dish sorting and collection system (1000), a plurality of proximity sensors (1101, 1102, 1103, 1104, 1105) and a control system (1200). For the purpose of this description and ease of understanding, the system (10) is explained herein with below reference to automatic feeding, identifying, counting, classifying, segregating and collecting organic samples such as but not limited to micro-organisms, where the organic sample is cultivated on petri dishes (D1, D2) in a microbiological laboratory. However, it is also within the scope of the invention to practice/implement the system (10) for automatic feeding, identifying, counting, classifying, segregating and collecting blood samples or specimens or any other organic samples taken from any living things in any of a research laboratory, food or beverage industry, pharmaceutical industry and any other applications without otherwise deterring the intended function of the system (10) as can be deduced from the description and corresponding drawings.

The petri dish feeding system (100) is adapted for holding and feeding the petri dishes (D1, D2). The petri dish feeding system (100) includes a plurality of first petri dish holding assemblies (102), a plurality of second petri dish holding assemblies (104), a petri dish feeding device (106), a petri dish locking device (108), a rotatable base (110) and a petri dish rotating system (114) and a coupler (116).

Each first petri dish holding assembly (102) is adapted to hold a plurality of first petri dishes (D1). For the purpose of of this description and ease of understanding, the first petri dish (D1) is considered to be 65 mm petri dish. It is also within the scope of the invention to provide the first petri dish (D1) in any other size. Each first petri dish holding assembly (102) includes a petri dish cassette (102C), a plurality of guide members (102R), a top support member (102H) a plurality of petri dish retention units (102L) and a plurality of covers (102HC). Each first petri dish holding assembly (102) is adapted to facilitate stacking of first petri dishes (D1) along a vertical direction. Each first petri dish holding assembly (102) can be easily docked and undocked with respect to the rotatable base (110) for ease in unloading and loading of first petri dishes (D1) thereof respectively. The petri dish cassette (102C) of each first petri dish holding assembly (102) is adapted to be docked onto the rotatable base (110) to facilitate unloading of the first petri dishes (D1) and also ensures accurate positioning. A portion of the petri dish cassette (102C) of each first petri dish holding assembly (102) is received by a cassette receiving portion (not shown) defined in the rotatable base (110). One end of each guide member (102R) is connected to the petri dish cassette (102C) and another end of each guide member (102R) is connected to the top support member (102H). The top support member (102H) is adapted to support the guide members (102R). The top support member (102H) is spaced away and opposite to the petri dish cassette (102C). Each cover (102HC) is adapted to cover corresponding petri dish retention unit (102L). For the purpose of this description and ease of understanding, each guide member (102H) is considered to be a guide rod. The petri dish retention units (102L) of each first petri dish holding assembly (102) is adapted to retain the first petri dishes (D1) in the first petri dish holding assembly (102) thereby restricting the first petri dishes (D1) from falling therefrom when the first petri dish holding assembly (102) is removed (undocked) from the rotatable base (110). The petri dish retention units (102L) of each first petri dish holding assembly (102) automatically releases the first petri dishes (D1) to the petri dish feeding device (106) when docked onto the rotatable base (110). In an embodiment, each petri dish retention unit (102L) includes a petri dish retention member (102LH) and a spring (102LS). The petri dish retention member (102LH) of each first petri dish locking unit (102L) is adapted to engage corresponding first petri dish (D1) thereby retaining the first petri dishes (D1) in the first petri dish holding assembly (102) when the first petri dish holding assembly (102) is undocked from the rotatable base (110). The petri dish retention member (102LH) is disengaged from the first petri dish (D1) thereby releasing the first petri dish (D1) from the first petri dish holding assembly (102) when the first petri dish holding assembly (102) is docked to the rotatable base (110). Each cover (102HC) is adapted to cover corresponding petri dish locking unit (102L).

Each second petri dish holding assembly (104) includes a petri dish cassette (104C), a plurality of guide members (104R), a top support member (104H), a plurality of petri dish retention units (104L) and a plurality of covers (104HC). Each second petri dish holding assembly (104) is adapted to hold a plurality of second petri dishes (D2). Each second petri dish holding assembly (104) is adapted to facilitate stacking of second petri dishes (D2) along a vertical direction. For the purpose of this description and ease of understanding, the second petri dish (D2) is considered to be 90 mm petri dish. It is also within the scope of the invention to provide the second petri dish (D2) in any other size. Each second petri dish holding assembly (104) can be easily docked and undocked with respect to the rotatable base (110) for ease in unloading and loading of second petri dishes (D2) therein.. The petri dish cassette (104C) of each second petri dish holding assembly (104) is adapted to be docked onto the rotatable base (110) to facilitate unloading of the second petri dishes (D2) and also ensures accurate positioning. A portion of the petri dish cassette (104C) of each second petri dish holding assembly (104) is received by a cassette receiving portion (not shown) defined in the rotatable base (110). One end of each guide member (104R) is connected to the petri dish cassette (104C) and another end of each guide member (104R) is connected to the top support member (104H). The top support member (104H) is adapted to support the guide members (104R). The top support member (104H) is spaced away and opposite to the petri dish cassette (104C). Each cover (104HC) is adapted to cover corresponding petri dish retention units (104L). For the purpose of this description and ease of understanding, each guide member (104H) is considered to be a guide rod. The petri dish retention units (104L) of each second petri dish holding assembly (104) automatically releases the second petri dishes (D2) to the petri dish feeding device (106) when docked on the rotatable base (110). The petri dish retention units (104L) of each second petri dish holding assembly (104) is adapted to retain the second petri dishes (D2) in the second petri dish holding assembly (104) thereby restricting the second petri dishes (D2) from falling therefrom when the second petri dish holding assembly (104) is removed (undocked) from the rotatable base (110). In an embodiment, each petri dish locking unit (104L) includes a petri dish retention member (104LH) and a spring (104LS). The petri dish locking member (104LH) of each petri dish locking unit (104L) is adapted to engage corresponding second petri dish (D2) thereby retaining the second pet dishes (D2) when the first petri dish holding assembly (102) is undocked from the rotatable base (110). and The petri dish locking member (104LH) of each petri dish locking unit (104L) is adapted to disengage from the second petri dish (D2) thereby releasing the second petri dish (D2) from the second petri dish holding assembly (104) when the second petri dish holding assembly (104) is docked to the rotatable base (110). Each cover (104HC) is adapted to cover corresponding petri dish retention unit (104LH). The plurality of first and second petri dish holding assemblies (102, 104) are docked onto the rotatable base (110) in an alternating manner.

The petri dish feeding device (106) is adapted to feed the petri dishes (D1, D2) received from one of the petri dish holding assemblies (102, 104) to a petri dish conveying member (404) of the petri dish conveying system (400). The petri dish feeding device (106) is an electric linear actuator device which includes an actuator (106M), an actuator mounting bracket (106MB), a plurality of movable members (106S, 106N, 106R), a coupler (106C), a connecting member (106CP), a support member (106SH), a limit switch (not shown), a limit switch mount (not shown), a guide rail holding member (106RH), a petri dish feeding member (106P), and a plurality of linear bearings (not shown). The actuator (106M) is adapted to move the petri dish feeding member (106P) through the plurality of movable members (106S, 106N, 106R). The actuator (106M) includes a controller unit adapted to be provided in communication with the master controller unit (not shown) of the control system (1200). For the purpose of this description and ease of understanding, the actuator (106M) is considered to be a stepper motor. The actuator mounting bracket (106MB) is adapted to mount the actuator (106M). The plurality of movable members (106S, 106N) includes a rotatable member (106S), a movable follower (106N) and a guide rail (106R). The rotatable member (106S) is adapted to guide a movement of the movable follower (106N). The rotatable member (106S) is rotatably coupled to the motor (106M) through the coupler (106). For the purpose of this description and ease of understanding, the rotatable member (106S) is considered to be a lead screw and correspondingly the movable follower (106N) is considered to be a lead screw nut. The coupler (106C) is adapted to couple the rotatable member (106S) to a shaft (not shown) of the actuator (106M). The connecting member (106CP) is adapted to connect the petri dish feeding member (106P) to the movable follower (106N). One end of the connecting member (106CP) is connected to the movable follower (106N) and another end of the connecting member (106CP) is connected to the petri dish feeding member (106P). The holder (106SH) is adapted to support one end of the rotatable guiding member (106S). The limit switch (not shown) is adapted to sense the position of movable follower (106N) or the petri dish feeding member (106P) and communicates the measured information to the master controller unit of the control system (1200). Accordingly, the master controller unit of the control system (1200) sends an input to a controller unit of the actuator (106M) thereby de-actuating the actuator (106M) for restricting a movement of the petri dish feeding member (106P) beyond a predefined position. The limit switch mount (not shown) is adapted to mount the limit switch. The petri dish feeding member (106P) feeds the petri dishes (D1, D2) received from one of the petri dish holding assemblies (102, 104) to the petri dish conveying member (404) of the petri dish conveying system (400). The master controller unit (not shown) activates the controller unit of the actuator (106M) only when one of the petri dish holding assemblies (102, 104) is in line with petri dish receiving portion (hole) of the petri dish conveying member (404) of the petri dish conveying system (400). The petri dish feeding member (106P) is actuated by the ball screw which is driven by the actuator (106M). The guide rail (106R) is adapted to guide a movement of the petri dish feeding member (106P). The guide rail holding member (106RH) is adapted to hold one end of the guide rail (106R). The petri dish feeding member (106P) is mounted onto the connecting member (106CP). The petri dish feeding member (106P) is adapted to feed the petri dishes (D1, D2) from one of the petri dish holding assemblies (102, 104) to the petri dish conveying member (104) of the petri dish conveying system (400). The plurality of linear bearings (not shown) is adapted to support the guide rail (106R). It is also within the scope of the invention to consider the petri dish feeding device (106) as one of a mechanical linear actuator, electro-pneumatic linear actuator, electro-hydraulic linear actuator, solenoid operated linear actuator, telescopic linear actuator, ball screw linear actuator, any other type of electric linear actuators and any other type of linear actuators.

The petri dish locking device (108) is adapted to lock the first petri dish (D1) of the first petri dish holding assembly (102). The petri dish locking device (108) is an electric linear actuator device which includes a locking member (108L), a rack gear (108RG) defined on one side of the locking member (108L), a pinion gear (108PG), a plurality of guide rails (108GR), an actuator (108M) and a main frame (108MF). The petri dish locking device (108) is a sliding lock and release mechanism mounted horizontally above the petri dish conveying member (404) of the petri dish conveying system (400). When the petri dish feeding system (100) moves to a petri dish feeding position, the locking member (108L) of the petri dish locking device (108) slides forward and holds the second last petri dishes in position while the bottom most petri dish is feed to the petri dish conveying member (404) of the petri dish conveying system (400).The locking member (108L) is adapted to be moved by the actuator (108M) between one of a locked position in which the locking member (108L) is engaged with the corresponding first petri dish (D1) thereby locking the first petri dish (D1) and an unlocked position in which the locking member (108L) is disengaged from the corresponding first petri dish (D1). The locking member (108L) defines a petri dish receiving portion (108LR) adapted to receive the second last petri dish (D1) when the locking member (108L) is in the locked position. The locking member (108L) is movably connected to the guide rails (108GR). The pinion gear (108PG) is rotatably coupled to the actuator (108M). The pinion gear (108PG) is engaged with the rack gear (108RG) of the locking member (108L). The plurality of guide rails (108GR) is adapted to guide a movement of the locking member (108L). One end of each guide rail (108GR) is connected to one end of the main frame (108MF) and another end of the guide rail (108GR) is connected to another end of the main frame (108MF). The actuator (108M) includes a controller unit adapted to be provided in communication with the master controller unit of the control system (1200). The actuator (108M) is adapted to move the locking member (108L) between one of the locked position and the unlocked position when the controller unit of the actuator (108M) receives input from the master controller unit of the control system (1200). For the purpose of this description and ease of understanding, the actuator (108M) is considered to be a servo motor. The main frame (108MF) is adapted to mount the actuator (108M) and the guide rails (108GR) thereby mounting the locking member (108L) thereof. It is also within the scope of the invention to consider the petri dish locking device (108) as one of a mechanical linear actuator, electro-pneumatic linear actuator, electro-hydraulic linear actuator, solenoid operated linear actuator, telescopic linear actuator, ball screw linear actuator, any other type of electric linear actuators and any other type of linear actuators.

The rotatable base (110) is adapted for holding the plurality of first and second petri dish holding assemblies (102, 104). The rotatable base (110) is rotated by the petri dish rotating system (114) in accurate steps of 45 degree each and hence 2 steps for reaching a quarter rotation.

The petri dish rotating system (114) is adapted to rotate the plurality of first and second petri dish holding assemblies (102, 104) through the rotatable base (110) and the coupler (116). The petri dish rotating system (114) includes an actuator (114M), a geneva (114G), a geneva cam (114C), a geneva coupler (114GC), a limit switch (114L), a limit switch mount (114LM), a drive shaft (114S), a plurality of bearing housings (114BH), a plurality of spacers (114SP) and a rotatable base connector (114RC). The actuator (114G) is adapted to rotate the first and second petri dish holding assemblies (102, 104) through the geneva (114G) and drive shaft (114S). For the purpose of this description and ease of understanding, the actuator (114M) is considered to be a motor. The geneva coupler (114GC) is adapted to couple the geneva (114G) to the drive shaft (114S). The geneva cam (114C) is rotatably engaged with the geneva (114G). The geneva (114G) is coupled to the drive shaft (114S) through corresponding the geneva coupler (114GC). The limit switch (114S) is adapted to sense the position of geneva cam (114C) and communicates the measured information to the master controller unit of the control system (1200). Accordingly, the master controller unit of the control system (1200) sends an input to a controller unit of the actuator (114M) thereby de-actuating the actuator (114M) for restricting a rotation of the first and second petri dish holding assemblies (102, 104) beyond a predefined position. The limit switch mount (114LM) is adapted to mount the limit switch (114L). One end of the drive shaft (114S) is coupled to the geneva (114G) and another end of the drive shaft (114S) is coupled to the plurality of first and second petri dish holding assemblies (102, 104) through the coupler (116). The rotatable base connector (114RC) is adapted to connect the drive shaft (114S) to the rotatable base (110). The coupler (116) is adapted to couple the drive shaft (114S) of the petri dish rotating system (114) to the top support member (102H, 104H) of the first and second petri dish holding assemblies (102, 104). It is also within the scope of the invention to provide any other mechanisms instead of Geneva mechanism (114G, 114C) for driving the drive shaft (114S) on operation of the actuator (114M).

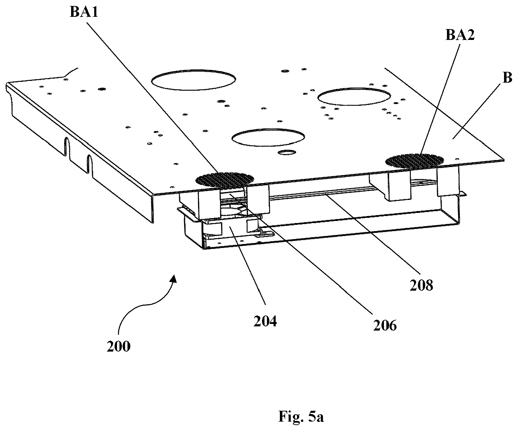

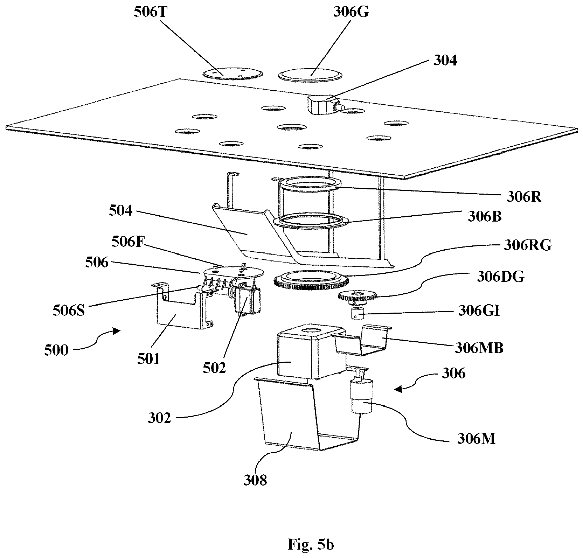

The temperature regulating system (200) is adapted to maintain the temperature of the organic samples cultivated on the petri dishes (D1, D2). The temperature regulating system (200) includes an enclosure (202), a fan (204), a heater (206) and a duct (208). The enclosure (202) is adapted to enclose the first and second petri dish holding assemblies (102, 104) of the petri dish feeding system (100). The enclosure (202) includes a rear wall, a plurality of side walls, a top wall and a movable front door. The fan (204) is positioned below the heater (206). The fan (204) is adapted to circulate heat air to the enclosure (202) through air vents (BA1) defined on a stationary base plate (B), as shown in fig. 5). The heater (206) is adapted to heat the air which is circulated by the fan (204) thereby maintaining the temperature of the organic samples at a predefined temperature. For example, the heater (206) is adapted to maintain a temperature of the organic sample(s) between 35 and 37 degree centigrade. The duct (208) is adapted to allow heat air flow to another end of the enclosure (202) thereby regulating temperature of organic samples. One end of the duct (208) is adapted to receive heat air which is circulated by the fan (204) and another end of the duct (208) is adapted to discharge heat air to another end of the enclosure (202) through another air vents (BA2) defined on the stationary base plate (B).

The petri dish identification system (300) is adapted to identify the petri dishes (D1, D2). The petri dish identification system (300) includes a first identifying device (302), a second identifying device (304), a petri dish rotating device (306) and a mounting bracket (308). For the purpose of this description and ease of understanding, the first and second identifying device (302, 304) are considered to be but not limited to a barcode scanner or QR code scanner, where the first identifying device (302) is a bottom barcode scanner and the second identifying device (304) is a side barcode scanner. The first and second identifying devices (302, 304) are adapted to identify the petri dishes (D1, D2) based on the barcode sticker or printed codes on the petri dishes (D1, D2). The barcode sticker or printed codes or QR code on the petri dishes (D1, D2) defines the composition, date of infusion, location and other details of the organic sample cultivated on the petri dishes (D1, D2). The first identifying device (302) is mounted on the mounting bracket (308). The petri dish rotating device (306) is adapted to facilitate the first and second identifying devices (302, 304) to identify the petri dishes (D1, D2). The petri dish rotating device (306) includes a motor (306M), a driving gear (306DG), a driven gear (306RG), a gear insert (306GI), a mounting bracket (306MB), a base member (306B), a rotatable member (306R) and a transparent member (306G). The motor (306M) is mounted onto the mounting bracket (306MB). The driving gear (306DG) is mounted onto a shaft of the motor (306M). The gear insert (306GI) is adapted to connect the driving gear (306DG) to the output shaft of the motor (306M). The driven gear (306RG) is rotatably connected to the driving gear (306DG). The base member (306B) is adapted to hold the rotatable member (306R) and the transparent member (306G). The rotatable member (306R) is rotatably mounted on the driven gear (306RG). The transparent member (306G) is rotatably mounted on the rotatable member (306R). The transparent member (306G) is adapted to facilitate identification of the petri dishes (D1, D2) by the first identification device (302). The motor (306M) rotates the transparent member (306G) through the rotatable member (306R) and the gears (306DG, 306RG) thereby enabling the first and second identifying devices (302, 304) to identify the petri dishes (D1, D2). The mounting bracket (308) is adapted to mount the first identifying device (302) thereon. In another embodiment, the examples of at least one of the first and second identifying device (302, 304) is considered to be but not limited to a radio frequency identification (RFID) device, near field communication (NFC) based identification device, Bluetooth low energy (BLE) based identification device and so on. In another embodiment, the petri dishes (D1, D2) can also be geo tagged for tracking of petri dish (D1, D2) in a controlled regulated environment.

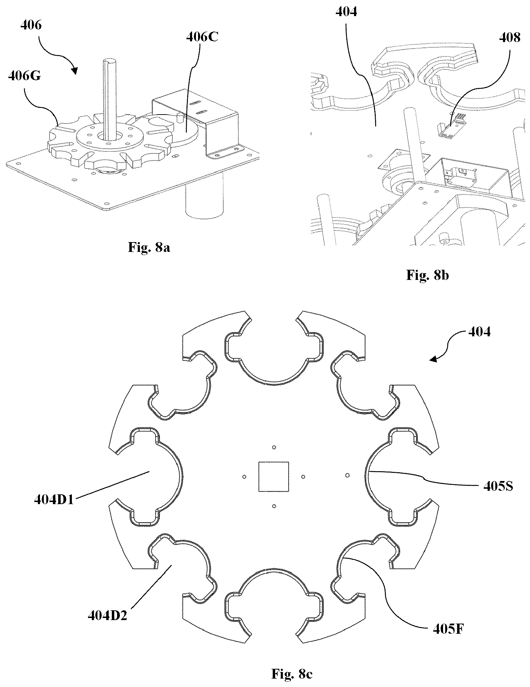

The petri dish conveying system (400) is adapted to convey the petri dishes (D1, D2) between the petri dish feeding system (102) and the petri dish sorting and collection system (1000). The petri dish conveying system (400) includes an actuator (402), a petri dish conveying member (404), a plurality of petri dish sleeves (405F, 405S), as shown in fig. 8c), a geneva(406G), a geneva cam (406C), a position sensor (408), a limit switch (409), a limit switch mounting bracket (410), a geneva coupler (412), a drive shaft (414), a plurality of bearing housings (416) and a drive shaft coupler (418). The actuator (402) is adapted to rotate the petri dish conveying member (404) through the drive shaft (414) and the geneva (406G). For the purpose of this description and ease of understanding, the actuator is considered to be a stepper motor. The petri dish conveying member (404) defines a plurality of petri dish receiving portions (404D1, 404D2), where the plurality of petri dish receiving portions (404D1, 104D2) includes a plurality of first petri dish receiving portions (404D1) and a plurality of second petri dish receiving portions (404D2). The first and second petri dish receiving portions (404D1, 404D2) are defined in the petri dish conveying member (404) in an alternate manner. For the purpose of this description and ease of understanding, each first and second petri dish receiving portion (404D1, 404D2) is considered to be a slot. The petri dish conveying member (404) is made of acrylic material. The petri dish conveying member (404) is adapted to convey the petri dishes (D1, D2) between the petri dish feeding system (102) and the petri dish sorting and collection system (1100). The petri dish conveying member (404) allow encapsulating the petri dishes and a stationery base plate (B) onto which the petri dishes rest and provide a slide-on surface. The distance between these two plates is accurately maintained to allow good transfer of petri dishes between petri dish feeding station, and petri dish sorting and collection station. The petri dish conveying member (404) is mounted on the metal chassis and is bounded by steel balls contraption to ensure the petri dish conveying member (404) rotates only in one plane and do not wobble. The geneva cam (406C) is rotatably connected to a shaft of the actuator (402). The geneva (406G) and the geneva cam (406C) are adapted to facilitate centric alignment of the petri dishes (D1, D2) with respect to each of the petri dish feeding system (100), the petri dish identifying system (300), the petri dish rejecting system (500), the scanning device (700) and the petri dish sorting and collection system (1000). The position sensor (408) is adapted to detect the position of the petri dish conveying member (404) and communicates the measured position of the petri dish conveying member (404) to a master controller unit of the control system (1200). The limit switch (409) is adapted to sense the position of geneva cam (406C) and communicates the measured position of geneva cam (406C) to the master controller unit of the control system (1200). Accordingly, the master controller unit of the control system (1200) sends an input to a controller unit of the actuator (402) thereby de-actuating the actuator (402) for restricting a rotation of the petri dish conveying member (404) beyond a predefined position. The limit switch mounting bracket (410) is adapted for mounting the limit switch (408). The geneva coupler (412) is adapted for coupling the geneva (406G) to the drive shaft (414). One end of the drive shaft (414) is coupled to the geneva (406G) through geneva coupler (412) and another end of the drive shaft (414) is coupled to the petri dish conveying member (404) through the drive shaft coupler (418). The drive shaft coupler (418) is adapted for coupling the drive shaft (414) to the petri dish conveying member (404). It is also within the scope of the invention to provide any other mechanisms instead of Geneva mechanism (406G, 406C) for driving the drive shaft (414) on operation of the actuator (402).

The petri dish rejecting system (500) is adapted to reject and collect the petri dishes (D1, D2) which are not identified by the identifying devices (302, 304). The petri dish rejecting system (500) includes a main frame (501), a motor (502), a petri dish guide member (504), a flap assembly (506), a coupler (not shown) and a petri dish collection station (508), (as shown in fig. 1). The flap assembly (506) includes a flap (506F) a flap top member (506T) and a flap shaft (506S). The flap top member (506T) is adjustably mounted onto the flap (506F). The motor (502) is adapted to move the flap assembly (506) between an open position in which the flap assembly (506) conveys the petri dishes (D1, D2) to the petri dish guide member (504) and a closed position in which the flap assembly (506) allows the petri dish conveying member (404) to move the petri dishes (D1, D2) to the scanner device (700). The flap shaft (506S) is rotated by the motor (502) to move the flap (506F) between the open position and the closed position. A portion of the flap shaft (506S) is inserted into a shaft receiving portion (not shown) of the flap (506F). The coupler (not shown) is adapted to couple the flap shaft (506F) to a shaft (not shown) of the motor (502). The petri dish guide member (504) is adapted to guide and facilitate movement of the petri dishes (D1, D2) to the petri dish collection station (500S) when the flap assembly (506) is in the open position. The petri dish collection station (508) is adapted to collect the petri dishes (D1, D2) which are not identified by the identifying devices (302, 304) of the petri dish identification system (300).

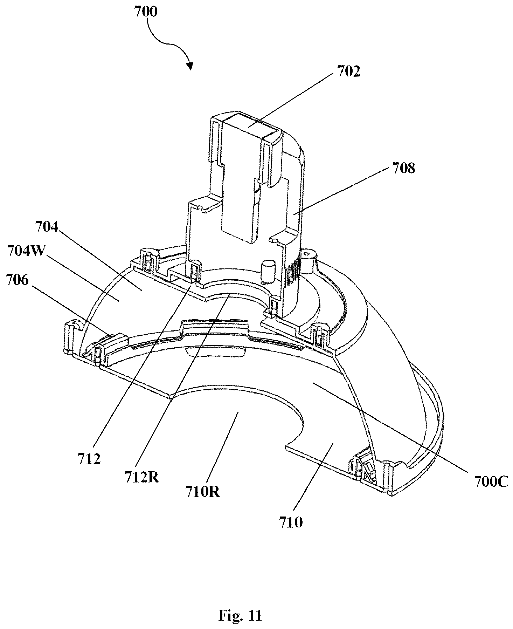

The scanner device (700) includes an image capture device (702), a light reflector (704), a plurality of lights (706), a light support ring (707), a holder (708), a first light diffuser (710) and a second light diffuser (712). The image capture device (702) is adapted to capture image(s) of the organic sample(s) based on input from an artificial intelligence (AI) based controller system (not shown). The image capture device (702) is disposed above the stationary light reflector (704). The image capture device (702) is mounted on the holder (708). The image capture device (702) is adapted to be moved to one of a plurality of positions in relation to the organic sample(s). For the purpose of this description and ease of understanding, the image capture device (702) is considered to be a camera. Examples of the image capture device (702) includes but not limited to digital camera, multispectral camera, charge coupled device (CCD) type camera, scanner, a thermal camera, an ultraviolet (UV) camera, near-infrared (NIR) camera and so on. However, it is also within the scope of the invention to use any other type of cameras for capturing the images of organic sample(s) without otherwise deterring the intended function of the image capture device (702) as can be deduced from the description and corresponding drawings. The image(s) captured by the image capture device (702) is transferred to the master controller unit of the control system (1200), where the master controller unit is an artificial intelligence (AI) based controller unit of the control system (1200). The AI based controller system provides an output on type of micro-organism present in the organic sample(s) and number of colonies present in each type of micro-organism based on the image(s) captured by the image capture device (702). It is also within the scope of the invention to configure the image capture device (702) to capture and transfer videos of the organic sample(s) to the AI based controller unit for determining the type of micro-organism present in the organic sample(s) and number of colonies present in each type of micro-organism. The AI based controller unit of the control system (1200) provides the output on type of micro-organisms present in the organic sample(s) and number of colonies present in each type of micro-organism to the user interface unit (800). In an embodiment, the stationary light reflector (704) is adapted to reflect the illumination of the lights (706) to facilitate uniform distribution of illumination to at least one of a photo compartment (700C), as shown in fig. 11) and the organic sample(s). The stationary light reflector (704) is always stationary in relation to the organic sample(s). The stationary light reflector (704), the first light diffuser (710) and the second light diffuser (712) defines the photo compartment (700C). An entirety of inner portion of the stationary light reflector (704) is coated with white color. In an embodiment, the stationary light reflector (704) substantially defines a dome shape configuration. In another embodiment, at least one of an inner portion and an outer portion the stationary light reflector (704) defines a polygonal shape configuration. It is also within the scope of the invention to provide the stationary light reflector (704) in any other shape without otherwise deterring the intended function of the stationary light reflector (704) as can be deduced from the description and corresponding drawings.

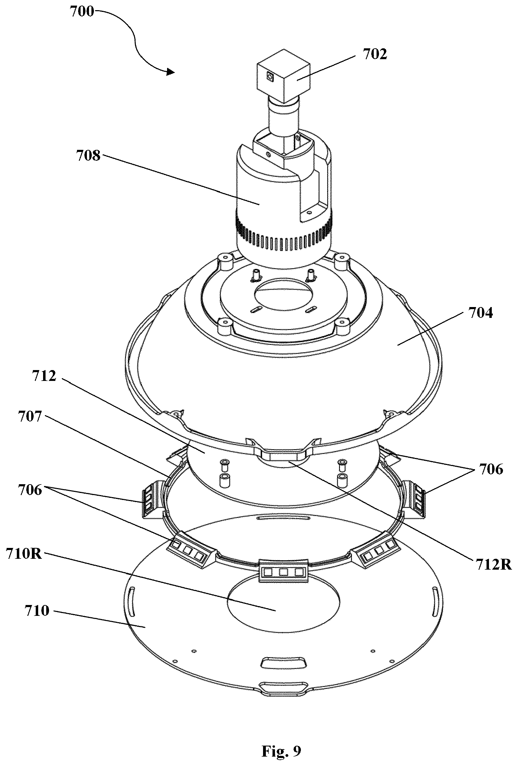

In an embodiment, the plurality of lights (706) is adapted to focus an illumination onto the stationary light reflector (704). The plurality of lights (706) is provided within the stationary light reflector (704). The plurality of lights (706) comprises at least one red light, at least one green light and at least one blue light. Each light (706) is a LED light. Each light (706) is near to and facing an inner wall (704W), as shown in fig. 11) of the stationary light reflector (704). The plurality of lights (706) is mounted on the light support ring (707). The lights (706) and the light support ring (707) are disposed on the first light diffuser (710), (as shown in fig. 9). The plurality of lights (706) is positioned in a circular array (as shown in fig. 9). The master controller unit of the control system (1200) is adapted to control the illumination level of the lights (706) by altering the intensity of the lights (706).

The holder (708) is adapted to hold the image capture device (702). In an embodiment, the holder (708) is adapted to facilitate a change in focus of the image capture device (702). The holder (708) is mounted on the stationary light reflector (704). In an embodiment, the first light diffuser (710) is adapted to diffuse the illumination of the lights (706) thereby reducing the reflection and glare of the illumination. The first light diffuser (710) defines at least one aperture (710R), as shown in fig. 9 and fig. 11) adapted to facilitate the image capture device (702) to capture image(s) of the organic sample therethrough. The organic sample cultivated on the petri dish is positioned below the aperture (710R) of the first light diffuser (710) and accordingly, the image capture device (702) captures images of the organic samples. The first light diffuser (710) is secured at a bottom end of the stationary light reflector (704). For example, the first light diffuser (710) is secured to the stationary light reflector (704) by using fasteners. It is also within the scope of the invention to secure the first light diffuser (710) to the stationary light reflector (704) by using any other temporary joint or permanent j oint. The first light diffuser (710) is provided below and spaced away and opposite to the second light diffuser (712). At least a portion of the first light diffuser (710) which is facing the second light diffuser (712) is coated with matte black to diffuse the illumination of the lights (706) thereby reducing the reflection and glare of the illumination emitted by the lights (706).

In an embodiment, the second light diffuser (712) is adapted to diffuse the illumination of the lights (706) thereby reducing the reflection and glare of the illumination. The second light diffuser (712) defines at least one aperture (712R), as shown in fig. 9 and fig. 11) adapted to facilitate the image capture device (702) to capture image(s) of the organic sample therethrough. The second light diffuser (712) is provided below and spaced away from the image capture device (702). The second light diffuser (712) is provided above and spaced away from the first light diffuser (710). The second light diffuser (712) is parallel and co-axial and opposite to the first light diffuser (710). The second light diffuser (712) is secured at a top end of the stationary light reflector (704). For example, the second light diffuser (712) is secured to the stationary light reflector (704) by using fasteners. It is also within the scope of the invention to secure the second light diffuser (712) to the stationary light reflector (704) by using any other temporary joint or permanent joint. At least a portion of the second light diffuser (712) which is facing the first light diffuser (710) is coated with matte black to diffuse the illumination of the lights (706) thereby reducing the reflection and glare of the illumination emitted by the lights (706).

The conditioning of light is achieved by using the stationary light reflector (704) and the light diffusers (710, 712). The plurality of lights (706), the stationary light reflector (704) and the light diffusers (710, 712) provides optimal lighting condition in the photo compartment (700C).

The user interface unit (800) is adapted to communicate the user defined inputs to the master controller unit (not shown) of the control system (1200). The user interface unit (800) includes a display screen (802), as shown in fig. 1b) which displays the output information about the organic sample(s) received by a controller unit of the user interface unit (800) from the master controller unit of the control system (1200). The information or image displayed on the display screen (802) of the user interface unit (800) includes but not limited to the scanned image of the organic sample together with a dot or contour or bounding box automatically superimposed over each individual colony that has been counted and also displays color coding based on the class of micro-organism detected. The output information also includes digital count on the number of colonies present in each of the classes separately.

The petri dish sorting and collection system (1000) is adapted to sort and collect the petri dishes (D1, D2) based on the input from the master controller unit (not shown) of the control system (1200). In an embodiment, the petri dish sorting and collection system (1000) is an electric linear actuator system which includes an actuator (1002), a petri dish sorting member (1004), an exit hopper assembly (1006), a petri dish guiding member (1007) and a collection bin (1008), a plurality of movable members (1010L, 1010N, 1010R), coupler (1012), a connecting member (1014), a stationary support rail (1016), an actuator mounting bracket (not shown), a limit switch (not shown), a limit switch mount (not shown), a guide rail support member (not shown) and a lead screw support member (not shown). The actuator (1002) is adapted to move the petri dish sorting member (1004) through the plurality of movable members (1010L, 1010N, 1010R). The actuator (102) includes a controller unit adapted to be provided in communication with the master controller unit (not shown) of the control system (1200). For the purpose of this description and ease of understanding, the actuator (1002) is considered to be a stepper motor. The actuator mounting bracket (not shown) is adapted to mount the actuator (1002). The plurality of movable members (1010L, 1010N, 1010R) includes a rotatable member (1010L), a movable follower (1010N) and a movable guide rail (1010R). The rotatable member (1010L) is adapted to guide a movement of the movable follower (1010N). The rotatable member (1010L) is rotatably coupled to the actuator (1002) through the coupler (1012). For the purpose of this description and ease of understanding, the rotatable member (1010L) is considered to be a lead screw and correspondingly the movable follower (1010N) is considered to be a lead screw nut. The coupler (1012) is adapted to couple the rotatable member (1010L) to a shaft (not shown) of the actuator (1002). The connecting member (1014) is adapted to connect the petri dish sorting member (1004) to the movable follower (1010N) through the movable guide rail (1010R). One end of the connecting member (1014) is connected to the movable follower (1010N) and another end of the connecting member (1014) is connected to the movable guide rail (1010R). The lead screw support member (not shown) is adapted to support one end of the rotatable member (1010L). The limit switch (not shown) is adapted to sense the position of movable follower (1010N) or the petri dish sorting member (1004) and communicates the measured information to the master controller unit of the control system (1200). Accordingly, the master controller unit of the control system (1200) sends an input to a controller unit of the actuator (1002) thereby de-actuating the actuator (1002) for restricting a movement of the petri dish sorting member (1004) beyond a predefined position. The limit switch mount (not shown) is adapted to mount the limit switch (not shown). The petri dish sorting member (1004) feeds the petri dishes (D1, D2) received from the petri dish conveying member (404) of the petri dish conveying system (400) to one of the exit hopper assembly (1006) or the collection bin (1008) based on the input sent by the master controller unit of the control system (1200) to the actuator (1002) of the petri dish sorting and collection system (1000). The movable guide rail (1010R) is adapted to guide a movement of the petri dish sorting member (1004). The stationary support rail (1016) is adapted to support the movable guide rail (1010R) and the connecting member (1014). The petri dish sorting member (1004) is mounted onto the movable guide rail (1010R). The exit hopper assembly (1006) is adapted to collect the petri dishes (D1, D2) in which microbial colonies are present in the organic samples. The exit hopper assembly (1006) includes an enclosure (1006E) and a plurality of holding forks (1006F). Each holding fork (1006F) is pivotally connected to the stationary base plate (B). The plurality of holding forks (1006F) is adapted to hold the petri dishes (D1, D2) in which microbial colonies are present in the organic samples. The plurality of holding forks (1006F) is adapted to facilitate stacking of the petri dishes (D1, D2) in a vertical manner. The petri dish guiding member (1007) is adapted to convey the petri dish (D1, D2) from the petri dish sorting member (1004) to the collection bin (1008). The collection bin (1008) is adapted to collect the petri dishes (D1, D2) in which microbial colonies are not present in the organic samples. It is also within the scope of the invention to consider the petri dish sorting and collection system (1000) as one of a mechanical linear actuator, electro-pneumatic linear actuator, electro-hydraulic linear actuator, solenoid operated linear actuator, telescopic linear actuator, ball screw linear actuator, any other type of electric linear actuators and any other type of linear actuators.

The plurality of proximity sensors (1101, 1102, 1103, 1104, 1105) includes a first proximity sensor (1101), a second proximity sensor (1102), a third proximity sensor (1103), a fourth proximity sensor (1104) and a fifth proximity sensor (1105). The first proximity sensor (1101) is adapted to detect the type of petri dish (D1, D2) of petri dish holding assembly (102, 104) and sends the sensed information to the master controller unit of the control system (1200). The second proximity sensor (1102) adapted to detect the presence of petri dish (D1, D2) in corresponding petri dish holding assembly (102, 104) and sends the sensed information to the master controller unit of the control system (1200). The first and second proximity sensors (1101, 1102) are located in vicinity of the petri dish locking device (108) of the petri dish feeding system (100). The third proximity sensor (1103) is adapted to detect the first petri dish slot of the petri dish conveying member (404) in vicinity of scanner device (700) based on a first identification element (407F) provided on the petri dish conveying member (404). For the purpose of this description and ease of understanding, the first identification element (407F) is considered to be an infra-red sticker. Accordingly, the third proximity sensor (1103) sends the sensed information to master controller unit of control system (1200). The fourth proximity sensor (1104) adapted to detect the second petri dish slot of the petri dish conveying member (404) in vicinity of scanner device (700) based on the identification element (407S) provided on the petri dish conveying member (404). For the purpose of this description and ease of understanding, the second identification element (407S) is considered to be an infra-red sticker. Accordingly fourth proximity sensor (1104) sends the sensed information to master controller unit of control system (1200). The fifth proximity sensor (1105) adapted to detect the position of of petri dish (D1, D2) which is positioned below aperture (710R) of first light diffuser (710) of scanner device (700) and accordingly fifth proximity sensor (1105) sends the sensed information to master controller unit of control system (1200). The third, fourth and fifth proximity sensors (1103, 1104, 1105) are located in vicinity of scanner device (700). It is also within the scope of the invention to use any other type of presence detecting sensors for type of the petri dish (D1, D2), position of the petri dish (D1, D2), presence of the petri dishes (D1, D2) and the type of petri dish slot on petri dish conveying member (404).

The control system (1200) is in communication with the user interface unit (800). The master controller unit (not shown) of the control system (1200) is in communication with the motor controller units of each of the petri dish feeding device (106), petri dish locking device (108), the petri dish rotating device (306), the petri dish conveying system (400) and the petri dish rejecting system (500). Further, the master controller unit of the control system (1200) is in communication with the position sensor (408) of the petri dish conveying system (400) and the image capture device (702) of the scanner device (700). The control system (1200) includes a data storage unit (not shown) adapted to store the data of the organic samples cultivated on the petri dishes (D1, D2).

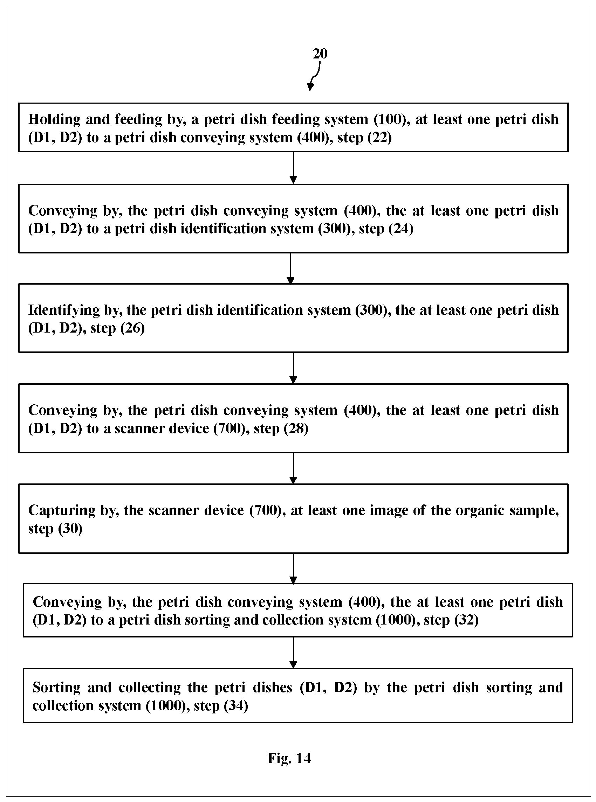

Fig. 14 depicts a flowchart a method (20) for automatic management of organic samples, according to embodiments as disclosed herein. For the purpose of this description and ease of understanding, the method (20) is explained herein below with reference to automatic feeding, identifying, counting, classifying, segregating and collecting organic samples such as but not limited to micro-organisms, where the organic sample is cultivated on petri dishes (D1, D2). However, it is also within the scope of this invention to practice/implement the entire steps of the method (20) in a same manner or in a different manner or with omission of at least one step to the method (20) or with any addition of at least one step to the method (20) for automatic feeding, identifying, counting, classifying, segregating and collecting blood samples or specimens or any other organic samples taken from any living things in any of a research laboratory, food or beverage industry, pharmaceutical industry and any other applications without otherwise deterring the intended function of the method (20) as can be deduced from the description and corresponding drawings. The method (20) comprises, holding and feeding, by a petri dish feeding system (100), at least one petri dish (D1, D2) to a petri dish conveying system (400), step (22).

At step 24, the method (20) includes conveying, by the petri dish conveying system (400), the at least one petri dish (D1, D2) to a petri dish identification system (300).

At step 26, the method (20) includes identifying, by the petri dish identification system (300), the at least one petri dish (D1, D2).

At step 28, the method (20) includes conveying, by the petri dish conveying system (400), the at least one petri dish (D1, D2) to a scanner device (700).

At step 30, the method (20) includes capturing, by the scanner device (700), at least one image of the organic sample.

At step 32, the method (20) includes conveying, by the petri dish conveying system (400), the at least one petri dish (D1, D2) to a petri dish sorting and collection system (1000).

At step 34, the method (20) includes sorting and collecting the petri dishes (D1, D2) by the petri dish sorting and collection system (1000).

Further, the method (20) comprises rejecting and collecting, by a petri dish rejecting system (500), the petri dishes (D1, D2) which are not identified by the petri dish identification system (300).

Further, the method (20) comprises providing by, the master controller unit of the control system, output on type of micro-organisms present in the organic sample(s) and number of colonies present in each type of micro-organisms to a user interface unit (800) based on the image(s) captured by scanner device (700).

Further, the method (20) comprises regulating, by a temperature regulating system (200), a temperature of organic samples cultivated on the petri dishes (D1, D2).

The method step of conveying, by the petri dish conveying system (400) between the petri dish feeding system (100) and the petri dish sorting and collection system (1000) comprises rotating, by an actuator (402) of the petri dish conveying system (400), a petri dish conveying member (404) through a geneva (114G) and a drive shaft (414).

The method step of holding and feeding, by a petri dish feeding system (100), at least one petri dish (D1, D2) to a petri dish conveying system (400) comprises,
holding, by at least one first petri dish holding assembly (102), the plurality of first petri dishes (D1);
holding, by at least one second petri dish holding assembly (104), the plurality of second petri dishes (D2);
moving, by an actuator (108M), a locking member (108L) of a petri dish locking device (108) in one of a locked position in which the locking member (108L) is engaged with corresponding petri dish (D1) and an unlocked position in which the locking member (108L) is disengaged from the petri dish (D1);
rotating, by an actuator (114M) of a petri dish rotating system (114), the plurality of first and second holding assemblies (102, 104) through a geneva (114G), a drive shaft (114S) and a rotatable base (110);
moving, by an actuator (106M), a petri dish feeding member (106P) of a petri dish feeding device (106) of said petri dish feeding system (100) in a direction towards said petri dish holding assembly (102, 104); and
feeding, by a petri dish feeding member (106P), the petri dish (D1, D2) received from one of the first petri dish holding assembly (102) or the second petri dish holding assembly (104) to the petri dish conveying member (404) of the petri dish conveying system (400).

The method step of sorting and collecting the petri dishes (D1, D2) by the petri dish sorting and collection system (1000) comprises,
moving, by an actuator (1002), a petri dish sorting member (1004) through a plurality of movable members (1010L, 1010N, 1010R);
moving, by the petri dish sorting member (1004), the petri dish (D1, D2) received from a petri dish conveying member (404) of said petri dish conveying system (400) to one of an exit hopper assembly (1006) or a collection bin (1008);
collecting, by the exit hopper assembly (1006), the petri dishes (D1, D2) in which microbial colonies are present in the organic samples as detected by master controller unit of the control system (1200);
guiding and conveying, by a petri dish guiding member (1007), the petri dishes (D1, D2) to the collection bin (1008); and
collecting by, the collection bin (1008), the petri dishes (D1, D2) in which microbial colonies are not present in the organic samples as detected by the master controller unit of the control system (1200).

The method step of capturing by, the scanner device (700), at least one image of the organic sample comprises,
focusing, by a plurality of lights (706), an illumination onto a stationary light reflector (704);
reflecting, by the stationary light reflector (704), the illumination of the lights (706) to facilitate uniform distribution of illumination to at least one of a photo compartment (700C) and the organic sample(s);
diffusing, by a first light diffuser (710) and a second light diffuser

(712), the illumination of the lights (706) to reduce the reflection and glare of the illumination from the plurality of lights (706);
positioning the organic sample(s) below at least one aperture (710R) of the first light diffuser (710);
capturing, by an image capture device (702), image(s) of the organic sample(s); and
sending the captured images of organic sample(s) to the master controller unit of the control system (1200).

The technical advantages of the system (10) for automatic management of organic samples are as follows. The system (10) automatically feeds, identifies, counts, classifies, segregates and collects organic samples such as but not limited to micro-organisms. The system (10) is modular automated colony counter system which can classify colonies in different classes such as bacteria and fungus present in organic sample and also gives a digital count on the number of colonies present in each of the classes separately. The system (10) is a compact automated colony counter system which consumes less space in the room. The system (10) provides geographical tracking of petri dishes (D1, D2) in a controlled regulated environment. The scanner device (700) of the system (10) automatically lightens conditions and captures accurate images of organic sample of cultivated on petri dishes (D1, D2) of different sizes. The scanner device (700) of the system (10) standardizes the imaging process by eliminating entrance of any ambient light by providing a closed photo compartment at all times thereby enhancing the quality of image captured by the device resulting in reliable colony counting. The system (10) is configured for automatic entry and exit of the organic sample(s) for point accuracy. The system is configured for automatic detection of the petri-dish inside a photo compartment and automatically triggers the scan acquisition control. The system (10) is a micro-biological colony counter which can display the scanned image of organic sample together with a dot or contour or bounding box automatically superimposed over each individual colony that has been counted and also displays color coding based on the class of micro-organism detected. The system (10) is micro-biological colony counter which provides an output on a digital count of the number of colonies such that the output can further be re-classified into different classes of micro-organisms by a trained technician. The system (10) is a micro-biological colony counter which can count surface colonies in petri-dish and can count colonies in different size or diameter of petri-dish. The system (10) is an automated micro-biological colony counter which reduces the time in counting and classification of microbes in each class separately, eliminates manual errors and requires less manual intervention. The system (10) is a automated micro-biological colony counter which is accurate, reliable and can perform the counting and classification of microbes in each class separately even in absence of well-trained technician.

The foregoing description of the specific embodiments will so fully reveal the general nature of the embodiments herein that others can, by applying current knowledge, readily modify and/or adapt for various applications such specific embodiments without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. Therefore, while the embodiments herein have been described in terms of embodiments, those skilled in the art will recognize that the embodiments herein can be practiced with modifications within the spirit and scope of the embodiments as described herein.

## Claims

1. A system for automatic management of organic sample(s), said system comprising:
a petri dish feeding system adapted for holding and feeding at least one petri dish;
a petri dish identification system adapted to identify the at least one petri dish;
a device adapted to capture images of the organic sample(s);
a petri dish sorting and collection system adapted to sort and collect the at least one petri dish;
a control system; and
a petri dish conveying system adapted to convey the at least one petri dish between said petri dish feeding system, and said petri dish sorting and collection system.

2. The system as claimed in claim 1, wherein said petri dish feeding system comprises,
a plurality of first petri dish holding assemblies, where each of said first petri dish holding assembly is adapted to hold a plurality of first petri dishes; and
a plurality of second petri dish holding assemblies, where each of said second petri dish holding assembly is adapted to hold a plurality of second petri dishes, where each of said first and second petri dish holding assembly comprises,
a petri dish cassette;
a top support member disposed opposite to said petri dish cassette;
a plurality of guide members, where one end of each of said guide member is connected to said petri dish cassette and another end of each of said guide member is connected to said top support member;
a plurality of petri dish retention units adapted to retain the plurality of petri dishes;
a temperature regulating system adapted to regulate temperature of organic sample(s), where said temperature regulating system comprises,
an enclosure adapted to enclose said first and second petri dish holding assemblies (of said petri dish feeding system;
a fan adapted to circulate heat air to said enclosure; and
at least one heater adapted to heat the air which is circulated by said fan to said enclosure thereby regulating the temperature of the organic sample(s); and
a rotatable base adapted for holding said plurality of first and second petri dish holding assemblies,
wherein
said petri dish retention assembly of each of said first and second petri dish holding assembly includes a plurality of petri dish retention members and a plurality of springs;
each of said petri dish retention member of said petri dish locking units of said first and second petri dish holding assemblies is adapted to engage corresponding petri dish thereby retaining the petri dishes when said first and second petri dish holding assemblies is undocked from the rotatable base; and
each of said petri dish retention member of said petri dish locking units of said first and second petri dish holding assemblies is adapted to disengage from corresponding petri dish thereby releasing the first petri dish when said first and second petri dish holding assemblies are docked to said rotatable base.

3. The system as claimed in claim 1, wherein said petri dish identifying system comprises,
a first identifying device adapted to identify the petri dish based on an identification element provided on a bottom portion of the petri dish and accordingly said first identifying device provides the identified information to a master controller unit of said control system; and
a mounting bracket adapted to mount said first identifying device thereon,
wherein
said first identifying device is at least a barcode scanner;
the identification element is one of a barcode sticker, printed code and quick response (QR) code provided on the bottom portion of the petri dish;
a petri dish rejecting system adapted to reject and collect the petri dishes which are not identified by said petri dish identification system, where said petri dish rejecting system comprises,
a motor;
a petri dish collection station;
a flap assembly, said flap assembly comprises a flap shaft (rotatably connected to said motor, a flap, said flap adapted to be connected to said flap shaft and a flap top member, said flap top member adjustably mounted onto said flap; and
a petri dish guide member,
wherein
said motor is activated by master controller unit of said control system and accordingly said motor is adapted to move said flap assembly to an open position in which said flap assembly conveys the petri dishes to said petri dish guide member when the petri dishes are not identified by said petri dish identification system;
said petri dish guide member is adapted to guide and facilitate a movement of the petri dishes to said petri dish collection station when said flap assembly is in the open position; and
said petri dish collection station adapted to collect the petri dishes from said petri dish guide member;
a second identifying device adapted to identify the petri dish based on an identification element provided on a side portion of the petri dish and accordingly said second identifying device provides the identified information to said master controller unit of said control system; and
a petri dish rotating device adapted to rotate the petri dish, said device comprising,
a motor;
a driving gear adapted to be rotatably connected to said motor;
a driven gear adapted to be rotatably connected to said driving gear;
a rotatable member adapted to be rotatably connected to said driven gear; and
a transparent member adapted to be rotatably mounted on said rotatable member,
wherein
said motor adapted to rotate said transparent member through said rotatable member and said gears thereby rotating the petri dish;
said transparent member allows said identifying devices to identify the petri dishes;
said second identifying device is at least a barcode scanner; and
the identification element is one of a barcode sticker and printed code provided on the side portion of the petri dish.

4. The system as claimed in claim 1, wherein said device comprises,
a user interface unit in communication with said master controller unit of said control system, where said user interface unit comprises a display screen adapted to display output information about the organic sample(s) received from said master controller unit, wherein said user interface unit is adapted to communicate user defined inputs to said master controller unit of said control system.
a stationary light reflector, said stationary light reflector is always stationary in relation to the organic sample(s);
a plurality of lights disposed within said stationary light reflector, said lights is adapted to focus an illumination onto said stationary light reflector;
a first light diffuser coupled to a bottom end of said stationary light reflector; a second light diffuser coupled to a top end of said stationary light reflector; an image capture device disposed above said second light diffuser;
a holder adapted to mount said image capture device onto said stationary light reflector; and
a light support ring adapted to mount said plurality of lights, wherein
each of said light is near to and facing an inner wall of said stationary light reflector;
said stationary light reflector substantially defines a dome shape configuration;
said image capture device is at least a camera;
said image capture device is adapted to be moved to one of a plurality of positions with respect to the organic sample(s);
said lights and said light support ring is disposed on said first light diffuser;
an inner portion of said light reflector is coated with white color;
at least a portion of said first light diffuser which is facing said second light diffuser is coated with matte black to diffuse the illumination of said lights thereby reducing the reflection and glare of the illumination of said lights;
a portion of said second light diffuser which is facing said first light diffuser is coated with matte black to diffuse the illumination of said lights thereby reducing the reflection and glare of the illumination of said lights;
said stationary light reflector, said first light diffuser and said second light diffuser defines a photo compartment;
said stationary light reflector is adapted to reflect the illumination of said lights to facilitate uniform distribution of illumination to at least one of the photo compartment and the organic sample(s);
said first and second light diffusers adapted to diffuse the illumination of said lights thereby reducing the reflection and glare of the illumination of said lights;
the organic sample(s) is positioned below at least one aperture of said first light diffuser;
said image capture device adapted to capture image(s) of the organic sample(s) based on input from said master controller unit of said control system;
said image capture device sends the captured images of organic sample(s) to said master controller unit of said control system; and
said master controller unit provides an output on type of micro-organisms present in the organic sample(s) and number of colonies present in each type of micro-organisms based on the image(s) captured by said image capture device.

5. The system as claimed in claim 1, wherein said petri dish feeding system comprises,
a petri dish rotating system adapted for rotating said plurality of first and second petri dish holding assemblies through said rotatable base, where said petri dish rotating system comprises,
an actuator;
a geneva cam adapted to be rotatably coupled to said actuator;
a geneva adapted to be rotatably connected to said geneva cam;
a drive shaft adapted to be coupled to said rotatable base, where one end of said drive shaft is rotatably coupled to said geneva and another end of said shaft is coupled to said top support member of said first and second petri dish holding assemblies through a coupler;
a geneva coupler adapted to couple said geneva to said drive shaft;
a limit switch adapted to sense the position of said geneva cam and communicates the measured position to said master controller unit of said control system, where said master controller unit is adapted to de-actuate said actuator thereby for restricting a rotation of said petri dish holding assemblies beyond a predefined position;
a limit switch mount adapted to mount said limit switch thereon; and
a rotatable base connector adapted to connect said drive shaft to said rotatable base, wherein
said actuator is at least a stepper motor and is adapted to rotate said first and second petri dish holding assemblies through said geneva and said drive shaft based on input received from said master controller unit of said control system.

6. The system as claimed in claim 5, wherein said petri dish feeding system comprises a petri dish locking device adapted for locking corresponding petri dish of corresponding said petri dish holding assembly, where said petri dish locking device comprises,
an actuator;
a locking member slidably mounted on at least one guide rail, said locking member defines a rack gear;
a pinion gear adapted to be movably connected to said rack gear, where said pinion gear is rotatably coupled to said actuator;
a first proximity sensor adapted to detect the petri dish and sends the sensed information to said master controller unit of said control system; and
a second proximity sensor adapted to detect the presence of petri dish in said petri dish holding assembly and sends the sensed information to said master controller unit of said control system,
wherein
said actuator is activated by said master controller unit of said control system and accordingly said actuator is adapted to move said locking member between one of a locked position in which said locking member is engaged with the corresponding petri dish thereby locking the petri dish and an unlocked position in which said locking member is disengaged from the corresponding petri dish; and
said first and second proximity sensors are located in vicinity of said petri dish locking device of said petri dish feeding system.

7. The system as claimed in claim 1, wherein said petri dish conveying system comprises,
an actuator;
a geneva cam adapted to be rotatably connected to said actuator;
a geneva adapted to be rotatably connected to said geneva cam;
a petri dish conveying member;
a drive shaft, where one end of said drive shaft is coupled to said geneva and another end of said drive shaft is coupled to said petri dish conveying member;
a position sensor adapted to detect the position of said petri dish conveying member and communicates the measured position of said petri dish conveying member to said master controller unit of said control system;
a limit switch adapted to sense the position of said geneva cam and communicates the measured information to said master controller unit of said control system, where said master controller unit is adapted to deactivate said actuator thereby restricting a rotation of said petri dish conveying member beyond a predefined position;
a limit switch mounting bracket adapted for mounting said limit switch;
a geneva coupler adapted for coupling the geneva to the drive shaft;
a plurality of first petri dish sleeves;
a plurality of second petri dish sleeves; and
a drive shaft coupler is adapted for coupling the drive shaft to the petri dish conveying member,
wherein
said actuator is adapted to rotate said petri dish conveying member through said geneva and said drive shaft based on input received from said master controller unit of said control system;
said actuator is at least a stepper motor;
said petri dish conveying member defines a plurality of first petri dish receiving portions and a plurality of second petri dish receiving portions;
each of said first petri dish receiving portions is adapted to receive corresponding first petri dish sleeve; and
each of said second petri dish receiving portion is adapted to receive corresponding second petri dish sleeve.

8. The system as claimed in claim 1, wherein said petri dish feeding system comprises a petri dish feeding device adapted to feed the petri dishes received from one of the petri dish holding assemblies to said petri dish conveying member of the petri dish conveying system, where said petri dish feeding system (comprises,
an actuator;
a plurality of movable members, which includes,
a rotatable member adapted to be rotatably coupled to said actuator, wherein said rotatable member is at least a lead screw;
a movable follower adapted to be movably connected to said rotatable member, wherein said movable follower is at least a nut;
a connecting member adapted to be connected to said movable follower; and
a guide rail adapted to be connected to said movable follower through said connecting member and said petri dish feeding member is mounted onto a top end of said guide rail; and
a petri dish feeding member adapted to be rotatably connected to said actuator through said plurality of movable members, which comprises
an actuator mounting bracket adapted to mount the actuator;
a limit switch adapted to sense the position of said movable follower or the petri dish feeding member and communicates the measured information to the master controller unit of the control system, where said master controller unit de-actuates the actuator thereby restricting a movement of the petri dish feeding member beyond a predefined position; and
a limit switch mount adapted to mount the limit switch, wherein
said actuator is adapted to move said petri dish feeding member through said movable members in a direction towards said petri dish holding assembly based on input from said master controller unit of said control system; and
said petri dish feeding member is adapted to feed the petri dish received from one of the first petri dish holding assembly or the second petri dish holding assembly to said petri dish conveying member of said petri dish conveying system.

9. The system as claimed in claim 8, wherein said system comprises a petri dish sorting and collection system adapted to sort and collect the petri dishes, where said petri dish sorting and collection system comprises,
an actuator;
a plurality of movable members, which comprises
a rotatable member adapted to be rotatably coupled to said actuator; a movable follower adapted to be movably connected to said rotatable member;
a movable guide rail adapted to be connected to said petri dish sorting member to said movable follower through a connecting member;
a limit switch adapted to sense the position of said movable follower or said petri dish sorting member and communicates the measured position to said master controller unit of the control system, where said master controller unit is adapted to deactivate said actuator thereby restricting a movement of said petri dish sorting member beyond a predefined position;
a limit switch mount adapted to mount said limit switch thereon; a stationary support rail adapted to support said movable guide rail or said connecting member; and
an actuator mounting bracket adapted to mount the actuator, wherein
said exit hopper assembly comprises an enclosure and a plurality of holding forks, said holding forks pivotally connected to a stationary base plate;
said petri dish sorting member is mounted onto said movable guide rail;
said rotatable member is at least a lead screw and correspondingly said movable follower is at least a nut; and
said actuator is at least a stepper motor;
a petri dish sorting member adapted to be movably connected to said actuator through said plurality of movable members;
an exit hopper assembly;
a petri dish guiding member; and
a collection bin,
wherein
said actuator is adapted to move said petri dish sorting member through said plurality of movable members based on input from said master controller unit of said control system;
said petri dish sorting member is adapted to move the petri dish received from said petri dish conveying member of said petri dish conveying system to one of said exit hopper assembly or said collection bin;
said exit hopper assembly is adapted to collect the petri dishes in which microbial colonies are present in the organic samples as detected by said master controller unit of said control system;
said petri dish guiding member is adapted to convey the petri dish received from said petri dish sorting member to said collection bin; and
said collection bin is adapted to collect the petri dishes in which microbial colonies are not present in the organic samples as detected by said master controller unit of said control system.

10. The system as claimed in claim 4, wherein said system comprises,
a third proximity sensor adapted to detect the first petri dish slot of said petri dish conveying member in vicinity of said scanner device and accordingly said third proximity sensor sends the sensed information to said master controller unit of said control system;
a fourth proximity sensor adapted to detect the second petri dish slot of said petri dish conveying member in vicinity of said scanner device and accordingly said fourth proximity sensor sends the sensed information to said master controller unit of said control system; and
a fifth proximity sensor adapted to detect the position of petri dish which is positioned below said aperture of said first light diffuser of said scanner device and accordingly said fifth proximity sensor sends the sensed information to said master controller unit of said control system,
where said third, fourth and fifth proximity sensors are located in vicinity of said scanner device.

11. A method for automatic management of organic samples cultivated on petri dishes, said method comprising:
holding and feeding, by a petri dish feeding system, at least one petri dish to a petri dish conveying system;
conveying, by a petri dish conveying system, the at least one petri dish to a petri dish identification system;
identifying, by the petri dish identification system, the at least one petri dish;
conveying, by the petri dish conveying system, the at least one petri dish to a scanner device;
capturing, by the scanner device, at least one image of the organic sample;
conveying, by the petri dish conveying system, the at least one petri dish to a petri dish sorting and collection system, which comprises of rotating, by an actuator of the petri dish conveying system, a petri dish conveying member through a geneva and a drive shaft;
sorting and collecting the petri dishes by the petri dish sorting and collection system; and
rejecting and collecting, by a petri dish rejecting system, the petri dishes which are not identified by the petri dish identification system.

12. The method as claimed in claim 11, said method comprises providing output on type of micro-organisms present in the organic sample(s) and number of colonies present in each type of micro-organisms to a user interface unit based on the image(s) captured by the scanner device.

13. The method as claimed in claim 11, wherein said method comprises regulating, by a temperature regulating system, a temperature of organic samples cultivated on the petri dishes.

14. The method as claimed in claim 11, wherein said holding and feeding, by a petri dish feeding system, at least one petri dish to a petri dish conveying system comprises,
holding, by at least one first petri dish holding assembly, the plurality of first petri dishes;
holding, by at least one second petri dish holding assembly, the plurality of second petri dishes;
moving, by an actuator, a locking member of a petri dish locking device in one of a locked position in which the locking member is engaged with corresponding petri dish and an unlocked position in which the locking member is disengaged from the petri dish;
rotating, by an actuator of a petri dish rotating system, the plurality of first and second holding assemblies through a geneva, a drive shaft and a rotatable base;
moving, by an actuator, a petri dish feeding member of a petri dish feeding device of said petri dish feeding system in a direction towards said petri dish holding assembly; and
feeding, by a petri dish feeding member, the petri dish received from one of the first petri dish holding assembly or the second petri dish holding assembly to a petri dish conveying member of said petri dish conveying system.

15. The method as claimed in claim 11, wherein sorting and collecting the petri dishes by the petri dish sorting and collection system comprises,
moving, by an actuator, a petri dish sorting member through a plurality of movable members;
moving, by the petri dish sorting member, the petri dish received from a petri dish conveying member of said petri dish conveying system to one of an exit hopper assembly or a collection bin;
collecting, by the exit hopper assembly, the petri dishes in which microbial colonies are present in the organic samples as detected by master controller unit of the control system;
guiding and conveying, by a petri dish guiding member, the petri dishes to the collection bin; and
collecting, by the collection bin, the petri dishes in which microbial colonies are not present in the organic samples as detected by the master controller unit of the control system.

16. The method as claimed in claim 11, wherein said capturing, by the scanner device, at least one image of the organic sample comprises,
focusing, by a plurality of lights, an illumination onto a stationary light reflector;
reflecting, by the stationary light reflector, the illumination of the lights to facilitate uniform distribution of illumination to at least one of a photo compartment and the organic sample(s);
diffusing, by a first light diffuser and a second light diffuser, the illumination of the lights to reduce the reflection and glare of the illumination by the plurality of lights;
positioning the organic sample(s) below at least one aperture of the first light diffuser;
capturing, by an image capture device, image(s) of the organic sample(s); and sending the captured images of organic sample(s) to the master controller unit of the control system.
